# NEUE EUROPÄISCHE PATENTSCHRIFT

(11) **EP 2 886 108 B2**
(45) Veröffentlichungstag und Bekanntmachung des Hinweises auf die Entscheidung über den Einspruch: **02.11.2022**
(45) Hinweis auf die Patenterteilung: 06.02.2019
(21) Anmeldenummer: 13199278.6
(22) Anmeldetag: 23.12.2013
(51) Int. Cl.: A61K 9/46, A61K 9/00, A61K 31/58, A61K 9/20

(54) **Optimierte pharmazeutische Formulierung zur Behandlung von entzündlichen Veränderungen des Ösophagus**
Optimised pharmaceutical formula for the treatment of inflammatory changes of the esophagus
Formulation pharmaceutique optimisée pour le traitement de modifications inflammatoires de l''sophage

(43) Veröffentlichungstag der Anmeldung: 24.06.2015
(73) Patentinhaber: Dr. Falk Pharma GmbH, 79108 Freiburg (DE)
(72) Erfinder: Greinwald, Roland, 79341 Kenzingen (DE); Müller, Ralph, 79111 Freiburg (DE); Pröls, Markus, 79100 Freiburg/Breisgau (DE); Wilhelm, Rudolf, 76476 Bischweier (DE)
(74) Vertreter: Lederer & Keller Patentanwälte Partnerschaft mbB

(56) Entgegenhaltungen:
- EP-A1- 1 595 533
- EP-A1- 2 151 235
- US-A1- 2007 020 330
- US-A1- 2010 034 894
- US-A1- 2011 123 460
- "1217 Tablet Breaking force" In: US Pharmacopeia 35: 1 May 2012 (2012-05-01), The United States Pharmacopeial Convention pages 868-870,
- DR. SCHLEUNIGER: "Key factors influencing measured tablet hardness", Pharmatron, 2011, pages 1-5, Retrieved from the Internet: URL:https://www.pharmaceutical-networking. com/wp-content/uploads/2012/06/Key-Factors -Influencing-Measured-Tablet-Hardness.pdf
- Anonymous: "WHT 3ME brochure. Fully Automated 4 in 1 Tablet Testing Instrument", THE PHARMA TEST GROUP, 1 April 2018 (2018-04-01), pages 1-13, Retrieved from the Internet: URL:https://www.pharma-test.de/wp-content/ uploads/2018/04/ptag-80-21000-wht-3me-e.pd f
- Rowe et al.: "HANDBOOK OF PHARMACEUTICAL EXCIPIENTS", 2006, London pages 53-55, 742-743,
- ACEVES et al.: "Oral Viscous Budesonide: A Potential New therapy for Eosinophilic Esophagitis in Children", Am J. Gastroenterol., vol. 102, no. 10, 2007, pages 2271-2279,
- DOHIL et al.: Gastroenterology, vol. 139, no. 2, 24 July 2010 (2010-07-24) , pages 418-429. e1,
- DILGER et al.: "Active Eosinophilic Esophagitis Is Associated with Impaired Elimination of Budesonide by Cytochrome P450 3A Enzymes", Digestion, vol. 87, no. 2, April 2013 (2013-04), pages 110-117,
- TAYEBI et al.: "Formulation and Evaluation of a Novel Matrix-Type Orally Disintegrating Ibuprofen Tablet", Iran J Pharm Res, vol. 10, no. 3, 2011, pages 469-479,
- KRISHNA et al.: "Treatment of Eosinophilic Esophagitis: Oral Viscous Budesonide Superior to Swallowed Fluticasone Spray?", Gastroenterol Hepatol., vol. 7, no. 1, January 2011 (2011-01), pages 55-59,

## Beschreibung

Für die Behandlung von entzündlichen Prozessen und Veränderungen der Speiseröhre, wie zum Beispiel der eosinophilen Ösophagitis, ist eine Arzneiform erforderlich, die nach oraler Anwendung die lokale Verfügbarkeit des Wirkstoffes Budesonid in genügend hoher Konzentration am Entzündungsort ermöglicht. Dieses als Ösophagus- bzw. SpeiseröhrenTargeting bezeichnete Konzept ist mit der oralen Applikation einer einfachen Wirkstofflösung nicht realisierbar, da ein hohes Risiko besteht, dass der Wirkstoff dann rasch und annährend quantitativ in den Magen geschluckt wird. Das Ziel des Ösophagus- bzw. Speiseröhrentargeting soll daher bevorzugt dadurch erreicht werden, dass ein langsames Entlanggleiten des Wirkstoffes an der Schleimhaut der Speiseröhre verbunden mit einer vollständigen Benetzung der Oberfläche sowie einer Anhaftung des Wirkstoffes ermöglicht wird. Diese Art der Anwendung bringt den Wirkstoff gezielt an den Wirkort. Weiterhin ist zu berücksichtigen, dass die therapeutische Behandlung der Speiseröhre idealerweise noch zusätzliche spezielle Anforderungen an die Arzneiform erfordert, je nachdem welche Patientenpopulation behandelt werden soll. Die vorliegende Erfindung ist speziell geeignet, eine altersgerechte Arzneiform zur Verfügung zu stellen, die bei erwachsenen Patienten einfach und verlässlich anzuwenden ist und somit eine hohe Einhaltungsquote der verschriebenen Tagesdosis ("Compliance") erreicht.

Die eosinophile Ösophagitis ist eine chronische, inflammatorische Erkrankung der Speiseröhre, die mit einer Funktionseinschränkung des Ösophagus (Speiseröhre) einhergeht und durch eine Infiltration des Speiseröhrenepithels mit eosinophilen Granulozyten gekennzeichnet ist. Seit Ende der '70er Jahre wurde die eosinophile Ösophagitis kasuistisch beschrieben und seit Ende der '90er Jahre vermehrt diagnostiziert. Es scheint eine durch Th2-Zellen vermittelte Antwort auf aerogene und auf durch Nahrungsmittel aufgenommene Allergene vorzuliegen, die zur Ausschüttung von IL-13, IL-5 und nachfolgend zur vermehrten Produktion von Eotaxin-3 führt. Hierdurch werden eosinophile Granulozyten angelockt. Klinisch stehen eine häufig lange bestehende Dysphagie, sowie Bolusimpaktationen im Vordergrund. Die Diagnosestellung erfordert standardgemäss den Nachweis von ≥ 15 Eosinophilen / high power field in der Speiseröhre bei Patienten mit Symptomen einer Funktionsstörung des Ösophagus. Bei manchen Patienten sind lediglich leicht zu übersehende Schleimhautveränderungen zu beobachten. Die eosinophile Ösophagitis befällt überwiegend Männer und tritt sowohl im Kindes-, wie auch im jüngeren Erwachsenenalter gehäuft auf.

Die WO 2009/064417 offenbart Zusammensetzungen für die Behandlung von gastrointestinalen Entzündungen. Die dort beschriebenen Zusammensetzungen umfassen ein Corticosteroid und wenigstens ein zusätzliches Mittel zur Behandlung der Entzündung. In der WO 2009/064457 werden Corticosteroid enthaltende Zusammensetzungen offenbart, die zur Behandlung von Entzündungen des Gastrointestinaltrakts geeignet sind. Die US 2007/0111978 beschreibt Verfahren zur Linderung von entzündlichen Erkrankungen des Gastrointestinaltraktes. Vorgeschlagen wird dort die Herstellung einer hochviskosen Lösung aus einer Budesonid-Suspension mit einer sehr hohen Konzentration an Sucralose. Die US 2009/0264392 beschreibt Verfahren und Zusammensetzungen, die geeignet sind zur Behandlung der eosinophilen Ösophagitis. Bei dieser Behandlungsmethode wird ein Steroid und ein an der Schleimhaut haftendes Mittel eingesetzt. EP2151235 discloses budesonide-containing effervescent tablets with aspartam. Dilger-K. Lazaro-L-L. Marx-C. Bussmann-C. Digestion. 2012. 110-11 reveals budesonide in use of treatment of eosinophilic esophagitis.

Es ist eine Aufgabe der vorliegenden Erfindung, pharmazeutische Formulierungen bereitzustellen, die einerseits Vorteile bei der Applikation mit sich bringen und die andererseits gut hergestellt und in einer lagerstabilen Form bereitgestellt werden können.

Die vorliegende Erfindung betrifft pharmazeutische Formulierungen, insbesondere eine Brausetablette zur orodispersiblen Anwendung laut Anspruch 1, die als pharmazeutischen Wirkstoff Budesonid oder ein pharmazeutisch verträgliches Salz oder Derivat davon umfasst und bevorzugt bei Erwachsenen zur Behandlung von entzündlichen Erkrankungen der Speiseröhre angewendet wird.

Allgemein bekannt ist die Art der Anwendung von Brausetabletten, die nach Auflösung in Wasser als Trink- oder Spüllösung angewendet werden. Diese Anwendung des Wirkstoffes hat für die beschriebene Indikation verschiedene Nachteile, wie beispielsweise die Verwendung eines größeren Volumens an Wasser (üblicherweise 250 ml) und die damit verbundene Verdünnung des Wirkstoffes bzw. die Verteilung des Wirkstoffes in der gesamten Mundhöhle sowie das üblicherweise praktizierte rasche Hinunterschlucken der Lösung in den Magen. Bekannt ist auch die Anwendung von Budesonid bei der eosinophilen Ösophagitis (Straumann et al., Gastroenterology, 2010, S. 1526-1537). Allerdings wurden in dieser Studie Flüssigampullen, sogenannte "Respules" verwendet, deren bestimmungsgemäßer Gebrauch die Inhalation ist, wobei der Ampulleninhalt vorab in einen Zerstäuber gefüllt und dann inhaliert wird. Abweichend von diesem bestimmungsgemäßen Gebrauch wurde in der zitierten Studie die Budesonidsuspension der Ampulle als Trinklösung angewendet.

Die erfindungsgemäße Brausetablette laut Anspruch 1 dagegen wird nicht vor der Anwendung in Wasser aufgelöst und grenzt sich daher deutlich von der üblichen Anwendungsweise der Brausetabletten ab. Die erfindungsgemäße Brausetablette wird nicht zur Bereitung einer Trinklösung verwendet, sondern wird oral angewendet und beispielsweise auf die Zunge, bevorzugt die Zungenspitze gelegt und löst sich langsam unter Speicheleinwirkung im Mund auf. Daher unterscheidet sich die erfindungsgemäße orodispersible Brausetablette deutlich von vorbekannten Formulierungen hinsichtlich Zusammensetzung und Form.

Die erfindungsgemäße orodispersible Brausetablette kann auch als linguale Brausetablette bezeichnet werden, da sie bei der Applikation bevorzugt in Kontakt mit der Zunge gebracht werden soll. Abgekürzt werden kann die Formulierungsform auch als BUL (= Budesonid-haltige linguale Brausetablette).

Die orodispersible Anwendung der Brausetablette ist eine spezielle Anwendungsform, bei der die Tablette als solche oral angewendet und zum Beispiel auf die Zunge, bevorzugt die Zungenspitze gelegt und nach Schließen des Mundes leicht gegen den Gaumen gepresst wird. Auf diese Weise kommt die Brausereaktion in Gang und der Speichelfluss wird innerhalb von ca. 5-15 Sekunden angeregt. Die Bestandteile der Tablette beginnen sich im Speichel aufzulösen. Durch den natürlichen Schluckreflex wird der Speichel dann portionsweise hinuntergeschluckt und benetzt kontinuierlich die Speiseröhre, so dass es auf diese Weise zu einer Anhaftung von Budesonid an der Schleimhaut kommt. Während des Zerfalls der Brausetablette schluckt der Patient durchschnittlich 5-10 mal, so dass die Speiseröhre permanent mit wirkstoffhaltiger Speichelflüssigkeit benetzt wird. Die Brausetablette zerfällt komplett, und es bleiben keine wahrnehmbaren Bruchstücke zurück. Ein Nachtrinken von Flüssigkeit wird nicht notwendig, da kein Reiz zu trinken ausgelöst wird. Ein Vorteil der optimierten Formulierung besteht also gerade darin, keine Flüssigkeit nachtrinken zu müssen, und damit eine ausreichend lange Verweildauer der therapeutisch aktiven Substanz Budesonid in den betroffenen Bereichen des Ösophagus zu gewährleisten. Die Anwendung ist nach 1,5 bis 2 Minuten abgeschlossen und ermöglicht eine konzentrierte Verteilung von Budesonid auf der Speiseröhre. Die Verteilung des Wirkstoffes innerhalb der Speiseröhre auf die Schleimhaut der Speiseröhre erfolgt dabei ausschließlich mit Speichel. Eine breite Verteilung von Budesonid bereits in der Mundhöhle wird ebenfalls vermieden. Die erfindungsgemäße orodispersible Brausetablette kann sehr bequem vom Patienten zu geeigneten Zeitpunkten eingenommen werden.

Die erfindungsgemäße Anwendung der Brausetablette reduziert deutlich das Risiko einer unerwünschten Aufnahme von Budesonid in den systemischen Kreislauf. So erfolgt keine buccale oder sublinguale Anwendung der Arzneiform. Es werden zudem beim bestimmungsgemäßen Gebrauch keine relevanten Budesonidmengen über die Mundschleimhaut aufgenommen. Gegenüber Schmelztabletten, die ebenfalls orodispersibel angewendet werden, besteht der eindeutige Vorteil, dass der Zerfall der Brausetabletten eine über 1,5 bis 2 Minuten dauernde kontinuierliche Freisetzung und damit topische Applikation des Wirkstoffes auf der Ösophagusschleimhaut erlaubt. Schmelztabletten besitzen für die Anwendung dagegen den Nachteil, dass sie sofort zerfallen und deshalb nur in einem Schluck genommen werden können. Zudem sind Brausetabletten im Vergleich zu Schmelztabletten einfacher industriell herzustellen.

Gegenstand der vorliegenden Erfindung sind daher orodispersible Brausetabletten enthaltend 0,4 bis 3 mg und ganz besonders bevorzugt 0,5 bis 2,0 mg Budesonid. Das eingesetzte Budesonid kann in jeder pharmazeutisch anwendbaren Form eingesetzt werden. Die angegebene Menge gibt die Gesamtmenge des Wirkstoffs Budesonid in einer orodispersiblen Brausetablette wieder. In bevorzugter Ausführungsform beinhaltet die erfindungsgemäße orodispersible Brausetablette keinen weiteren pharmazeutischen Wirkstoff.

Ein wesentlicher Bestandteil der orodispersiblen Brausetablette ist ein System, das in Gegenwart von Speichel Gas entwickeln kann, um so die Brausewirkung zu erzielen. Voraussetzung ist, dass dieses Gas entwickelnde System pharmazeutisch verträglich ist. Ein derartiges Gas entwickelndes System besteht einerseits aus einer schwachen Säure bzw. einem Salz einer schwachen Säure. In Gegenwart von Wasser entsteht hieraus die freie schwache Säure. Die Säure darf allerdings nicht zu stark sein, um gesundheitliche Beeinträchtigungen zu vermeiden. Es kann sich hierbei um Weinsäure, Essigsäure, Milchsäure und besonders bevorzugt um Zitronensäure handeln. Üblicherweise werden die Salze der Säuren eingesetzt, also beispielsweise Natrium-, Magnesium- oder Calciumsalze. Die andere Komponente des Gas entwickelnden Systems besteht aus einem Salz einer Säure, das im Zusammenwirken mit einer weiteren Säure ein Gas freisetzen kann. Bevorzugt handelt es sich um Salze der Kohlensäure. Hierbei kann es sich um Carbonate oder Hydrogencarbonate, wie beispielsweise Natrium- oder Kaliumcarbonat oder Natrium- oder Kaliumhydrogencarbonate oder Calciumcarbonate, sowie Mischungen dieser Salze handeln. Wesentlich ist, dass ein gesundheitlich unbedenkliches Gas, nämlich CO₂, in kleinen Mengen bei Anwendung der Brausetablette freigesetzt wird. Das Gas wird durch das Zusammenwirken der Gas erzeugenden Komponenten mit Speichel freigesetzt.

Eine erfindungsgemäße orodispersible Brausetablette weist eine Masse auf, die zwischen 100 mg und 200 mg beträgt, bevorzugt haben die orodispersiblen Brausetabletten eine Masse von 120 mg bis 160 mg. Ganz besonders bevorzugte Ausführungsformen der orodispersiblen Brausetabletten haben eine Masse von 133 mg bis 147 mg.

Die Abmessungen der erfindungsgemäßen Brausetablette sind ebenfalls wesentlich. Bevorzugt haben die Brausetabletten einen Durchmesser, der zwischen 5 und 10 mm liegt. Es ist bevorzugt, dass die Brausetabletten rund sind, wobei allerdings nicht zwangsläufig eine ganz runde Form erforderlich ist. Abweichungen von der runden Form können gewählt werden. Die Brausetabletten sind biplan, wobei der Durchmesser bevorzugt zwischen 6,0 und 8,0 mm und besonders bevorzugt zwischen 6,9 und 7,3 mm liegt. Die Höhe der orodispersiblen Brausetablette liegt bevorzugt zwischen 1,5 und 3,0 mm, besonders bevorzugt zwischen 1,6 und 2,8 mm und ganz besonders bevorzugt zwischen 1,8 und 2,6 mm.

Was die mechanischen Eigenschaften der orodispersiblen Brausetablette angeht, sind diese wesentlich. Die Bruchfestigkeit der Brausetablette liegt bevorzugt zwischen einem Wert von 10 und 100 N, besonders bevorzugt zwischen 20 N und 70 N und wird bevorzugt bestimmt nach der European Pharmacopea gemäß der Monographie 2.9.8.

Bei der Prüfung der mechanischen Festigkeit von Tabletten wird der feste Prüfling zwischen zwei Stempeln oder einem Stempel und einem Amboss eingespannt. Dann lässt man eine Kraft auf den Prüfling (Tablette) einwirken und ermittelt die Kraft, die erforderlich ist, um einen Bruch in der Tablette zu bewirken. Die Bestimmung der Bruchfestigkeit der erfindungsgemäßen orodispersiblen Brausetablette kann beispielsweise mit vollautomatischen Testvorrichtungen durch geführt werden. Solche Testsysteme werden beispielsweise von der Firma Pharmatest unter der Bezeichnung WHT 3ME angeboten. Selbstverständlich können auch gleichwertige Testgeräte eingesetzt werden.

Die bevorzugte Bruchfestigkeit ermöglicht einerseits eine gute und reproduzierbare industrielle Herstellung, aber auch eine ausreichende mechanische Stabilität. Erfindungsgemäß weisen die orodispersiblen Brausetabletten eine Friabilität (Abriebfestigkeit) von maximal 5 %, bevorzugt maximal 1 %, auf. Die Friabilität (Abriebfestigkeit) wird bevorzugt bestimmt nach der European Pharmacopea gemäß der Monographie 2.9.7.

Form, Konsistenz und mechanische Eigenschaften der orodispersiblen Brausetablette sind wesentlich, da Größe und Form einerseits den kontinuierlichen und verhältnismäßig gleichmäßigen Zerfall in Gegenwart von Speichel ermöglichen. Andererseits erlauben sie eine hinreichende mechanische Stabilität, so dass die Brausetablette bei der bestimmungsgemäßen Anwendung nicht vorzeitig in Teilstücke zerbricht und dadurch eine erfindungsgemäße Anwendung nicht mehr ermöglicht. Die Eigenschaften der orodispersiblen Brausetablette haben zur Folge, dass kein Fremdkörpergefühl im Mund entsteht, und somit die Anwendung als subjektiv angenehm von dem Patienten empfunden wird.

Die in der besonders bevorzugten Ausführungsform beschriebenen Eigenschaften der Brausetablette erlauben die variable Einzeldosisverabreichung von Budesonid in einer Menge von bevorzugt 0,5 bis 3 mg Budesonid. Die verarbeitete Menge an Wirkstoff hat dabei keinen Einfluss auf die beschriebenen und für die Anwendung relevanten Qualitätsparameter wie Größe, Form und mechanische Stabilität. Eine Teilung der Tablette ist nicht erforderlich, vielmehr entspricht eine Tablette einer Anwendungsdosis.

Je niedriger die Dosis von Budesonid in der Brausetablette, umso höher sind die Anforderungen an die Auswahl geeigneter Hilfsstoffe zur Gewährleistung einer ausreichenden Langzeitstabilität der Arzneiform. Ein unerwünschter Abbau des Wirkstoffes wäre dann besonders unerwünscht, wenn niedrige Dosierungen verabreicht werden, weil dann nicht mehr die pharmazeutisch ausreichende Menge des Wirkstoffes in den Tabletten vorhanden wäre. Überraschenderweise konnte gezeigt werden, dass die physikalisch-chemische Stabilität von Budesonid in der Brausetablette nur durch die erfindungsgemäße Zusammensetzung ermöglicht wird. Auch wenn einzelne Hilfsstoffe zu dem Stand der Technik von Brausetabletten gehören, ist nur mit genau der erfindungsgemäßen qualitativen und quantitativen Zusammensetzung eine Langzeitstabilität der Arzneiform von bis zu 36 Monaten für alle Dosisstärken einer Budesonid-Brausetablette zur orodispersiblen Anwendung möglich. Die erfindungsgemäßen Brausetabletten können bei Raumtemperatur gelagert werden.

In einer bevorzugten Ausführungsform beinhaltet eine erfindungsgemäße orodispersible Brausetablette ein Polyvinylpyrrolidon. Hierbei handelt es sich um Polymerisationsprodukte des Vinylpyrrolidons. Die niedermolekularen Polyvinylpyrrolidone sind hygroskopisch, besonders bevorzugt wird erfindungsgemäß das Povidon K25 eingesetzt. Es ist auch möglich, auf dem Gebiet bekannte Derivate von Polyvinylpyrrolidon einzusetzen. Die eingesetzte Menge an Polyvinylpyrrolidon (beispielsweise Povidon K25) beträgt bevorzugt 1 bis 10, besonders bevorzugt 1,5 bis 3,5 Gew.-% bezogen auf die fertige Tablette.

Es ist erforderlich, dass die erfindungsgemäße orodispersible Brausetablette einen angenehmen Geschmack aufweist. Daher wird in bevorzugter Ausführungsform ein Stoff zugesetzt, der einen süßen Geschmack beim Auflösen im Mund hinterlässt. Da die Verwendung von Saccharose nachteilig sein kann, wird bevorzugt ein alternatives Süßmittel eingesetzt. In bevorzugter Ausführungsform ist dies Sucralose. Bei Sucralose handelt es sich um Saccharose, wobei Hydroxylreste durch Chloratome ersetzt sind. Sucralose ist im Vergleich zu Sucrose deutlich süßer. Allerdings müssen die Herstellungsbedingungen und die anderen Bestandteile der orodispersiblen Brausetablette so abgestimmt sein, dass keine Zersetzung der Sucralose auftritt, die zu unerwünschten Verfärbungen führen könnte. In der orodispersiblen Brausetablette beträgt der Anteil an Sucralose bevorzugt zwischen 0,1 und 1,0 Gew.-%, besonders bevorzugt zwischen 0,2 und 0,6 Gew.-% bezogen auf die fertige Tablette.

Ein anderer bevorzugt eingesetzter Bestandteil der orodispersiblen Brausetablette ist Docusat-Natrium. Hierbei handelt es sich um eine weiße, wachsartige Substanz, die als Solubilisator und Emulgator insbesondere für den Wirkstoff Budesonid eingesetzt wird. Die Menge an Docusat-Natrium beträgt bevorzugt zwischen 0,01 und 0,2 Gew.-%, besonders bevorzugt 0,02 bis 0,15 Gew.-% bezogen auf die fertige Tablette.

Ein weiterer bevorzugt eingesetzter Hilfsstoff ist Mannitol. Bei dem erfindungsgemäß eingesetzten Mannitol handelt es sich in bevorzugter Ausführungsform um einen polymorphen kristallinen Feststoff. Die beiden am häufigsten charakterisierten Modifikationen sind β- und α-Mannitol, die auch als Modifikation I und II gekennzeichnet werden. Als weitere Modifikation wurde auch schon δ-Mannitol beschrieben. Für die Herstellung der erfindungsgemäßen orodispersiblen Brausetabletten müssen die Hilfsstoffeigenschaften, insbesondere des Mannitols, besonderen Anforderungen genügen. Einerseits ist erforderlich, dass die Pulvermischungen bei der Herstellung eine gute Fließfähigkeit aufweisen, andererseits ist eine optimale Kompaktibilität, also die Fähigkeit, Tabletten mit hoher Festigkeit bei geringer Presskraft zu bilden, wesentlich. Eine zentrale Rolle kann hierbei die Partikelgrößeverteilung der einzelnen Mannitolkristalle spielen. Erfindungsgemäß wird bevorzugt eine Menge von 2,0 bis 10,0 Gew.-%, besonders bevorzugt 4,0 bis 7,0 Gew.-% Mannitol bezogen auf die fertige Tablette eingesetzt.

Ein weiterer wichtiger Hilfsstoff, der erfindungsgemäß bevorzugt eingesetzt wird, sind die Polyethylen-Glykole. In einer bevorzugten Ausführungsform wird Macrogol 6000 in einer Menge von 1,0 bis 10 Gew.-%, bevorzugt 2,0 bis 7,0 Gew.-%, bezogen auf die fertige Tablette, eingesetzt.

Eine weitere, bevorzugte Hilfsstoffkomponente ist Magnesiumstearat, das bevorzugt in einer Menge von 0,01 bis 0,5 Gew.-%, besonders bevorzugt in einer Menge von 0,05 bis 0,15 Gew.-% eingesetzt wird.

Die Komponenten, die die Brausewirkung erzielen, machen gewichtsmäßig den Hauptteil der orodispersiblen Brausetablette aus. Hierbei handelt es sich in bevorzugter Ausführungsform um eine Mischung aus Dinatriumcitrat, Mononatriumcitrat sowie Natriumhydrogencarbonat. Diese Brausemischung macht einen Gewichtsanteil von etwa 70 bis 95 Gew.-%, bevorzugt 85 bis 92 Gew.-%, bezogen auf die fertige orodispersible Brausetablette aus. Vor allem die Partikelform der Komponenten der Brausemischung ist für die mechanischen Eigenschaften der orodispersiblen Brausetablette maßgeblich. Daher müssen aus den im Handel verfügbaren Qualitäten der einzelnen Komponenten solche ausgewählt werden, die im Zusammenwirken mit den anderen Hilfsstoffen und mit dem pharmazeutisch aktiven Wirkstoff Budesonid so verpreßt werden können, dass die Eigenschaften der fertigen orodispersiblen Brausetablette, insbesondere die angestrebte Bruchfestigkeit und Friabilität, erzielt werden können.

Selbstverständlich müssen sich alle Komponenten der orodispersiblen Brausetablette auf 100 Gew.-% addieren.

Die erfindungsgemäße orodispersible Brausetablette kann bevorzugt zur Therapie, aber auch zur Vorbeugung von entzündlichen Veränderungen der Speiseröhre eingesetzt werden. Die erfindungsgemäße Formulierung ermöglicht eine für den Patienten angenehme kontinuierliche Abgabe des Wirkstoffes, der verhältnismäßig gleichmäßig auf der Speiseröhre verteilt wird. Der Wirkstoff Budesonid wird somit gezielt und effizient topisch an die entzündlichen Orte der Speiseröhre verbracht. Besonders bevorzugt werden die erfindungsgemäßen orodispersiblen Brausetabletten zur Therapie von eosinophiler Ösophagitis verwendet.

Bevorzugte Ausführungsformen der vorliegenden Erfindung werden durch die nachfolgenden Beispiele verdeutlicht.

### Beispiel 1:

Überraschenderweise wurde gefunden, dass eine physikalisch-chemische Stabilität der Budesonid-Brausetabletten von bis zu 36 Monaten, sowie die für die Anwendung der Brausetablette relevanten Qualitätsparameter wie Größe, Form und mechanische Stabilität durch die in der **Tabelle 1** angegebene qualitative und quantitative Zusammensetzung erzielt werden kann.

In Tabelle 1 sind besonders bevorzugte Konzentrationsangaben der erfindungsgemäß bevorzugt eingesetzten Komponenten wiedergegeben. Die in der Tabelle wiedergegebenen Werte müssen nicht zwangsläufig völlig exakt eingehalten werden. Allerdings sind die wesentlichen Komponenten und die Verhältnisse der einzelnen Komponenten zueinander entscheidend für die vorteilhaften Eigenschaften der erfindungsgemäßen orodispersiblen Budesonid-Brausetabletten.

**Tabelle 1: Zusammensetzung von bevorzugten Budesonid-Brausetabletten**

| **Zusammensetzung [mg]** | | | |
|---|---|---|---|
| | Budesonid 0,5 mg Brausetablette | Budesonid 1 mg Brausetablette | Budesonid 2 mg Brausetablette |
| Granulat | | | |
| Budesonid | 0,50 | 1,00 | 2,00 |
| Dinatriumcitrat | 67,00 | 67,00 | 67,00 |
| Mononatriumcitrat | 15,00 | 15,00 | 15,00 |
| Natriumhydrogencarbonat | 45,00 | 45,00 | 45,00 |
| Povidon K25 | 2,00 | 2,00 | 2,00 |
| Sucralose | 0,30 | 0,30 | 0,30 |
| Docusat-Natrium | 0,05 | 0,05 | 0,05 |
| Summe | 129,85 | 130,35 | 131,35 |

| Endmischung | | | |
|---|---|---|---|
| Mannitol | 5,95 | 5,95 | 5,95 |
| Macrogol 6000 | 5,00 | 5,00 | 5,00 |
| Magnesiumstearat | 0,10 | 0,10 | 0,10 |
| Summe | 140,90 | 141,40 | 142,40 |

### Beispiel 2:

Die Ergebnisse der Haltbarkeitsuntersuchungen von Budesonid 1 mg Brausetabletten bei unterschiedlichen Lagerungsbedingungen sind in Tabelle 2 zusammengefasst. Im Vergleich zu den Startwerten zeigten sich selbst bei Lagerung unter den Bedingungen von Belastungsversuchen keine relevanten Veränderungen.

**Tabelle 2: Stabilitätsergebnisse von Budesonid 1 mg Brausetabletten**

| | Lagerungsdauer bei 25°C/60% relative Feuchte in Monaten | | | | | | |
|---|---|---|---|---|---|---|---|
| | Initial | 3 | 6 | 9 | 12 | 18 | 24 |
| Bruchfestigkeit [N] | 36 | 33 | 35 | 35 | 36 | 36 | 35 |
| Zerfall in Wasser [Minuten] | 1,3 | 1,4 | 1,3 | 1,8 | 1,5 | 1,4 | 2,9 |
| Gehalt Budesonid [%] | 101,4 | 99,5 | 100,0 | 100,7 | 99,5 | 99,9 | 98,0 |
| Summe an Gesamtverunreinigungen [%] | < 0,1 | 0,13 | 0,11 | 0,12 | 0,13 | 0,19 | 0,22 |

| | Lagerungsdauer bei 30°C/65% relative Feuchte in Monaten | | | | | | |
|---|---|---|---|---|---|---|---|
| | Initial | 3 | 6 | 9 | 12 | 18 | |
| Bruchfestigkeit [N] | 36 | 37 | 34 | 36 | 34 | 28 | |
| Zerfall in Wasser [Minuten] | 1,3 | 1,7 | 1,5 | 1,7 | 1,7 | 2,9 | |
| Gehalt Budesonid [%] | 101,4 | 100,5 | 99,6 | 100,0 | 98,1 | 97,3 | |
| Summe an Gesamtverunreinigungen [%] | < 0,1 | 0,13 | 0,12 | 0,15 | 0,18 | 0,40 | |

| | | Lagerungsdauer bei 40°C/75% relative Feuchte in Monaten | | | | | |
|---|---|---|---|---|---|---|---|
| | | Initial | 1 | 2 | 3 | | |
| Bruchfestigkeit [N] | | 36 | 21 | 36 | 38 | | |
| Zerfall in Wasser [Minuten] | | 1,3 | 0,9 | 1,7 | 1,8 | | |
| Gehalt Budesonid [%] | | 101,4 | 100,2 | 100,6 | 99,5 | | |
| Summe an Gesamtverunreinigungen [%] | | < 0,1 | 0,14 | 0,27 | 0,39 | | |

### Beispiel 3:

Im Vergleich zu den Startwerten zeigen sich vor allem bei Lagerung unter den Bedingungen der Klimazone II keine relevanten Veränderungen. Dagegen zeigten die Ergebnisse der Tabelle 3, dass bei nur geringfügiger Abweichung von dieser Rezeptur (z. B. Austausch von 0,40 mg Sucralose durch 1,0 mg Aspartam) die Langzeitstabilität der Budesonid 1 mg Brausetabletten nicht mehr gegeben ist. Wie der Fehlversuch eindrucksvoll offenlegt, erhöht sich bei abgeänderter Rezeptur der Abbau von Budesonid nach vergleichbar langen Lagerungszeiträumen etwa um einen Faktor von ca. 7. Da dieser Abbau bei Dosierungen von weniger als 1 mg Budesonid noch deutlicher ausfällt, ist die physikalisch-chemische Stabilität der Brausetablette bevorzugt mit der in Tabelle 1 angegebenen qualitativen und quantitativen Zusammensetzung gegeben. Bei weniger als 1 mg Budesonid per orodispersibler Brausetablette fällt die Instabilität noch deutlicher aus.

**Tabelle 3: Mangelnde Stabilität von Budesonid 1 mg Brausetabletten mit modifizierter Zusammensetzung**

| | Lagerungsdauer bei 25°C/60% relative Feuchte in Monaten | | | | | | |
|---|---|---|---|---|---|---|---|
| | Initial | 3 | 6 | 9 | 12 | 18 | 27 |
| Gehalt Budesonid [%] | 98,6 | 99,7 | 101,1 | 99,8 | 98,9 | 96,8 | 95,7 |
| Summe an Gesamtverunreinigungen [%] | < 0,1 | 0,31 | 0,66 | 0,86 | 0,81 | 1,05 | 1,63 |

| | | | Lagerungsdauer bei 30°C/65% relative Feuchte in Monaten | | | | |
|---|---|---|---|---|---|---|---|
| | | | Initial | 3 | 6 | 9 | 12 |
| Gehalt Budesonid [%] | | | 98,6 | 100,1 | 100,4 | 99,4 | 98,2 |
| Summe an Gesamtverunreinigungen [%] | | | < 0,1 | < 0,1 | 0,91 | 1,01 | 1,31 |

Bei diesem Versuch wurde 0,4 mg Sucralose durch 1,0 mg Aspartam ersetzt.

### Beispiel 4:

### Klinische Daten:

In einer 4-armigen doppel-blinden, randomisierten, placebo-kontrollierten Multicenter-Studie der Phase-II wurden 2x1mg/Tag oder 2x2mg/Tag Budesonid-Brausetabletten mit einer 2x2mg/Tag oralen viskosen Budesonid-Suspension oder Placebo bei der Behandlung von aktiver eosinophiler Ösophagitis verglichen. Die Verblindung wurde durch die Anwendung der "Double-Dummy"-Technik gewährleistet. Ziel der Studie war es, die Überlegenheit der Budesonidformulierungen gegenüber Placebo zu zeigen. Der erste primäre Teilendpunkt dieser Studie war die Rate der histologischen Remission, wobei die Eosinophilen (eos) der Patienten in Remission eine mittlere Zahl von <16eos/mm² hpf ("high power field", also das Gesichtsfeld im Mikroskop bei einer 400x Vergrößerung) nach 2 Wochen Behandlung erreichen mussten. Als zweiter primärer Teilendpunkt wurde die Differenz in der mittleren Zahl der eos/mm² hpf zwischen Studienstart und dem Ende der Behandlung gemessen. Beide beschriebenen Wirksamkeitsparameter wurden konfirmativ in einem geschlossenen Testverfahren überprüft, damit ein Vergleich aller drei Verumgruppen mit der Placebogruppe möglich wurde. Das Design dieser Studie einschließlich der beschriebenen Endpunkte ist nahezu identisch zur Studie, wie sie in der Publikation von Straumann, 2010 aaO beschrieben wurde.

Überraschenderweise waren die Ergebnisse aller drei Testformulierungen nicht nur signifikant besser als Placebo, sondern auch besser als die Budesonidformulierung, welche in der Arbeit von Straumann et al., 2010 aaO beschrieben ist, in der Dosierung 2x1mg/Tag ist dieses Ergebnis signifikant.

Tabelle 4 zeigt die Ergebnisse für die histologische Remission, definiert als im Mittel <16eos/mm² hpf. Patienten, die vorzeitig die Studie beendeten, ohne dass eine histologische Folgeuntersuchung stattgefunden hat wurden als Patienten, die nicht in Remission sind, analysiert. In einer Sensitivitätsanalyse wurden nur solche Patienten analysiert, die die Studie komplettiert hatten.

**Tabelle 4: Histologische Remission Anzahl (%) der Patienten, die eine histologische Remission aufwiesen**

| | **BUU-2/EEA** | | | **Straumann (2010 aaO)** | |
|---|---|---|---|---|---|
| | **Budesonid 2x1 mg Tablette** | **Budesonid 2x2 mg Tablette** | **Placebo** | **Budesonid 2x1 mg suspension** | **Placebo** |
| **FAS** | | | | | |
| At wk 12** | | | | | |
| (LOCF) ITT | 19/19 **(100%)** | 18/19 **(95%)** | 0/19 **(0%)** | 13/18 **(72%)** | 2/18 **(11%)** |
| [95% RCI] | [64.7%; 100%] | [57.6%; 99.5%] | --- | NA | --- |

| | Anzahl (%) der Patienten, die eine histologische Remission aufwiesen | | | | |
|---|---|---|---|---|---|
| | **BUU-2/EEA** | | | **Straumann (2010 aaO)** | |
| | **Budesonid 2x1 mg Tablette** | **Budesonid 2x2 mg Tablette** | **Placebo** | **Budesonid 2x1 mg suspension** | **Placebo** |
| **FAS** | | | | | |
| P-value* | **< 0.0001** | **< 0.0001** | --- | **< 0.0001** | --- |

| **PP** | | | | | |
|---|---|---|---|---|---|
| At wk 12** | | | | | |
| (LOCF) ITT | 19/19**(100%)** | 17/17 **(100%)** | 0/17 **(0%)** | --- | --- |
| [95% RCI] | [63.1%; 100%] | [61.3%; 100%] | --- | --- | --- |
| p-Wert* | < **0.0001** | < **0.0001** | --- | --- | --- |
| Histologische Remission definiert als < 5 eos/hpf (entsprechend <16 eos/mm² hpf) FAS, Full Analyse Set; PP, Per-Protokoll Analyse Set; RCI, Wiederholter Zuverlässigkeits-Intervall (für den Unterschied zwischen Verum und Placebo) | | | | | |
| *für Überlegenheit von Verum gegenüber Placebo | | | | | |
| **At wk 12, Bei 12 Wochen | | | | | |

Auch für die Analyse des co-primären Endpunktes konnte Überlegenheit gegenüber Placebo gezeigt werden (Tabelle 5). (Der Vergleich zu Straumann ist nicht möglich, da dieser Endpunkt bei Straumann nicht untersucht wurde).

**Tabelle 5: Differenz in der mittleren Zahl eos/mm² hpf (FAS)**

| | **Budesonid 1 mg Tablette (n = 19)** | **Budesonid 2 mg Tablette (n = 19)** | **Budesonid 2 mg Susp. (n = 19)** | **Placebo (n = 19)** |
|---|---|---|---|---|
| Durchschnitt (SD) | -119.919 (79.275) | -128.120 (78.457) | -96.665 (124.253) | -7.882 (157.939) |
| n | n = 19 | n = 18 | n = 18 | n = 19 |
| p-Wert * | **0.0006** | **0.0005** | **0.0041** | --- |
| *für Überlegenheit von Verum gegenüber Placebo | | | | |

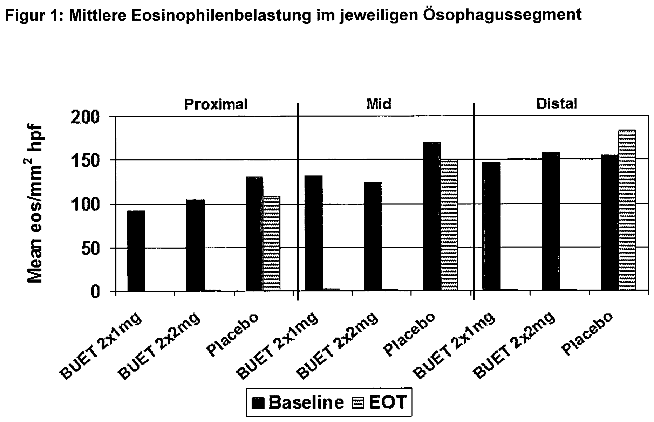

Zusätzlich konnte für alle Verumgruppen gezeigt werden, dass in allen Ösophagussegmenten die Eosinophilenbelastung nahezu komplett eliminiert werden konnte (in einen Bereich von 0,2-2,7 eos/mm² hpf). Dieses Ergebnis belegt, dass die erfindungsgemäße pharmazeutische Formulierung den Wirkstoff ösophagus-spezifisch über den gesamten Ösophagus verteilt. Dies ist in Figur 1 dargestellt.

Die Figur 1 zeigt die mittlere Eosinophilenbelastung in dem jeweiligen Ösophagussegment. Der Ösophagus wurde unterteilt in proximalen Teil, Mittelteil (Mid) und distalen Teil. Angegeben wurde die mittlere Eosinenzahl/mm² hpf. Die Abkürzung EOT bedeutet "End of Treatment".

### Beispiel 5:

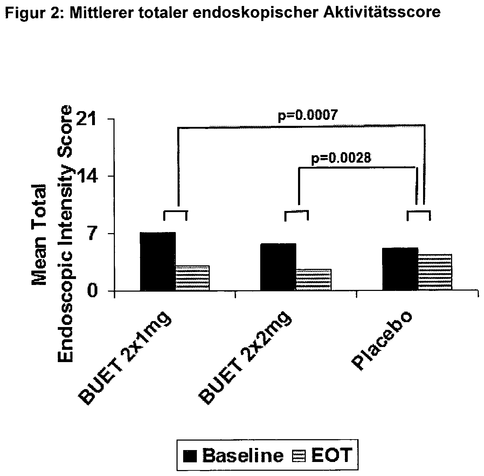

Neben der beschriebenen Wirksamkeit auf die Histologie konnte gezeigt werden, dass eine 2-wöchige Behandlung mit der erfindungsgemäßen Formulierung auch zu einer statistisch signifikanten und klinisch relevanten Verbesserung der eosinophilen Ösophagitis bezogen auf das endoskopische Bild führt. Dies ist in Figur 2 dargestellt.

In Figur 2 wird ein mittlerer endoskopischer Aktivitätsscore wiedergegeben. Die Abkürzung BUET bedeutet orodispersible, Budesonid enthaltende Brausetablette. Die Abkürzung 2x1 mg bedeutet zweimalige Gabe von je einer Tablette mit 1 mg Wirkstoff. Die Abkürzung 2x2 mg bedeutet die zweimalige tägliche Gabe einer orodispersiblen Brausetablette mit 2 mg Wirkstoff. Baseline bedeutet die Grundlinie, EOT bedeutet "End of Treatment". Hier bedeutet Baseline die erste Visite des Patienten, an der dieser mit der Behandlung beginnt und EOT ist die letzte Visite unter der Behandlung. Da immer das Prinzip des LOCF (Last observation carried forward) angewendet wird, kann die EOT-Visite zu unterschiedlichen Zeitpunkten stattfinden, aber immer mit allen Testparametern der protokollgemässen letzten Visite des Patienten.

### Beispiel 6:

Die erfindungsgemäßen orodispersiblen Brausetabletten mit 2 mg Budesonid pro Tablette wurden in einer Studie einer Gruppe von Patienten im Alter zwischen 20 und 50 Jahren verabreicht. Die Patienten wurden in einer Testgruppe behandelt mit einer 2 mg Budesonid-Tabelle pro Tag. Die Kontrollgruppe erhielt eine orodispersible Brausetablette ohne Wirkstoff (Placebo). Im Laufe einer 15-tägigen Behandlung wurde festgestellt, dass die orodispersible Budesonid enthaltende Brausetablette gut verträglich war und, dass sich die histologischen und klinischen Parameter deutlich verbesserten, im Gegensatz zu der Placebo-Gruppe. Die orodispersible Brausetablette wurde hinsichtlich der Anwendbarkeit von allen Probanden als durchweg positiv beurteilt.

## Patentansprüche

1. Orodispersible Brausetablette enthaltend 0,4 mg bis 3 mg Budesonid, das Salz wenigstens einer pharmakologisch akzeptablen Säure, die in wässriger Umgebung mit einer weiteren Säure ein Gas freisetzen kann, sowie das Salz einer weiteren schwachen Säure oder eine weitere schwache Säure, die in wässriger Lösung den pH-Wert erniedrigt und Sucralose in einer Menge von 0,1 bis 1,0 Gew.-% bezogen auf die fertige Tablette, **dadurch gekennzeichnet, dass** die biplane Brausetablette eine Masse von 100 mg bis 200 mg aufweist, einen Durchmesser von 6,0 bis 8,0 mm aufweist und eine Höhe von 1,6 bis 2,8 mm aufweist sowie eine Bruchfestigkeit von 10 N bis 100 N und eine Friabilität bestimmt nach der European Pharmacopoeia gemäß der Monographie 2.9.7 von maximal 5 % aufweist.

2. Orodispersible Brausetablette nach Anspruch 1, **dadurch gekennzeichnet, dass** das Salz der pharmazeutisch annehmbaren Säure, die in wässriger Umgebung mit einer Säure ein Gas freisetzen kann, NaHCO₃, Na₂CO₃, KHCO₃, K₂CO₂, CaCO₃, oder eine Mischung hiervon ist.

3. Orodispersible Brausetablette nach Anspruch 1 oder 2, **dadurch gekennzeichnet, dass** das Salz der weiteren pharmazeutisch akzeptablen schwachen Säure, das in wässriger Lösung den pH-Wert erniedrigt, Dinatriumcitrat, Mononatriumcitrat oder eine Mischung hiervon ist.

4. Orodispersible Brausetablette nach einem der vorhergehenden Ansprüche, **dadurch gekennzeichnet, dass** sie Povidon K25 in einer Menge von 0,5 bis 10 Gew.-% bezogen auf die fertige Tablette aufweist.

5. Orodispersible Brausetablette nach einem der vorhergehenden Ansprüche, **dadurch gekennzeichnet, dass** sie Docusat-Na in einer Menge von 0,01 bis 0,2 Gew.-% bezogen auf die fertige Tablette aufweist.

6. Orodispersible Brausetablette nach einem der vorhergehenden Ansprüche, **dadurch gekennzeichnet, dass** sie Mannitol in einer Menge von 2,0 bis 10,0 Gew.-% bezogen auf die fertige Tablette aufweist.

7. Orodispersible Brausetablette nach einem der vorhergehenden Ansprüche, **dadurch gekennzeichnet, dass** sie Macrogol 6000 in einer Menge von 1,0 bis 10,0 Gew.-% bezogen auf die fertige Tablette aufweist.

8. Orodispersible Brausetablette nach einem der vorhergehenden Ansprüche, **dadurch gekennzeichnet, dass** sie Magnesiumstearat in einer Menge von 0,05 bis 0,5 Gew.-% bezogen auf die fertige Tablette aufweist.

9. Orodispersible Brausetablette nach einem der vorhergehenden Ansprüche zur Verwendung bei der Behandlung von entzündlichen Veränderungen der Speiseröhre.

10. Orodispersible Brausetablette zur Verwendung nach Anspruch 9, wobei die entzündliche Veränderung eine eosinophile Ösophagitis ist.

## Claims

1. An orodispersible effervescent tablet containing 0,4 mg to 3 mg of budesonide, the salt of at least one pharmacologically acceptable acid that in an aqueous environment can release a gas with a further acid as well as the salt of a further weak acid or a further weak acid that decreases the pH value in an aqueous solution, and sucralose in an amount of 0.1 to 1.0% by weight, based on the finished tablet, **characterized in that** the biplane effervescent tablet has a mass of 100 mg to 200 mg, a diameter of 6,0 to 8,0 mm, and a height of 1,6 to 2,8 mm, as well as a fracture strength of 10 N to 100 N and a friability determined in accordance with the European Pharmacopoeia according to the monograph 2.9.7 of at most 5%.

2. The orodispersible effervescent tablet according to claim 1, **characterized in that** the salt of the pharmaceutically acceptable acid that in an aqueous environment can release a gas with an acid is NaHCO₃, Na₂CO₃, KHCO₃, K₂CO₂, CaCO₃, or a mixture thereof.

3. The orodispersible effervescent tablet according to claim 1 or 2, **characterized in that** the salt of the further pharmaceutically acceptable weak acid that decreases the pH value in an aqueous solution is disodium citrate, monosodium citrate or a mixture thereof.

4. The orodispersible effervescent tablet according to any one of the preceding claims, **characterized in that** it has povidone K25 in an amount of 0.5 to 10% by weight, based on the finished tablet.

5. The orodispersible effervescent tablet according to any one of the preceding claims, **characterized in that** it has docusate Na in an amount of 0.01 to 0.2% by weight, based on the finished tablet.

6. The orodispersible effervescent tablet according to any one of the preceding claims, **characterized in that** it has mannitol in an amount of 2.0 to 10.0% by weight, based on the finished tablet.

7. The orodispersible effervescent tablet according to any one of the preceding claims, **characterized in that** it has macrogol 6000 in an amount of 1.0 to 10.0% by weight, based on the finished tablet.

8. The orodispersible effervescent tablet according to any one of the preceding claims, **characterized in that** it has magnesium stearate in an amount of 0.05 to 0.5% by weight, based on the finished tablet.

9. The orodispersible effervescent tablet according to any one of the preceding claims for use in the treatment of inflammatory conditions of the esophagus.

10. The orodispersible effervescent tablet for use according to claim 9, the inflammatory condition being an eosinophilic esophagitis.

## Revendications

1. Comprimé effervescent orodispersible contenant 0,4 mg à 3 mg de budésonide et le sel d'au moins un acide pharmacologiquement acceptable, qui peut libérer un gaz dans un milieu aqueux avec un autre acide, ainsi que le sel d'un autre acide faible ou un autre acide faible, qui diminue le pH dans une solution aqueuse, et de la sucralose dans une quantité de 0,1 à 1% en poids par rapport au comprimé terminé, **caractérisé en ce que** le comprimé effervescent orodispersible biplan présente une masse de 100 mg à 200 mg, qu'il présente un diamètre de 6,0 à 8,0 mm et une hauteur de 1,6 à 2,8 mm de même qu'une résistance à la rupture de 10 N à 100 N et une friabilité déterminée selon la Pharmacopée Européenne en conformité avec la monographie 2.9.7 d'un maximum de 5 %.

2. Comprimé effervescent orodispersible selon la revendication 1, **caractérisé en ce que** le sel de l'acide pharmaceutiquement acceptable, qui peut libérer un gaz dans un milieu aqueux avec un acide, est NaHCO₃, Na₂CO₃, KHCO₃, K₂CO₂, CaCO₃, ou un mélange de ceux-ci.

3. Comprimé effervescent orodispersible selon la revendication 1 ou 2, **caractérisé en ce que** le sel de l'autre acide faible pharmaceutiquement acceptable, qui diminue le pH dans une solution aqueuse, est du citrate de disodium, du citrate de monosodium ou un mélange de ceux-ci.

4. Comprimé effervescent orodispersible selon l'une quelconque des revendications précédentes, **caractérisé en ce qu'**il présente de la povidone K25 dans une quantité de 0,5 à 10 % en poids par rapport au comprimé terminé.

5. Comprimé effervescent orodispersible selon l'une quelconque des revendications précédentes, **caractérisé en ce qu'**il présente du docusate de sodium dans une quantité de 0,01 à 0,2 % en poids par rapport au comprimé terminé.

6. Comprimé effervescent orodispersible selon l'une quelconque des revendications précédentes, **caractérisé en ce qu'**il présente du mannitol dans une quantité de 2,0 à 10,0 % en poids par rapport au comprimé terminé.

7. Comprimé effervescent orodispersible selon l'une quelconque des revendications précédentes, **caractérisé en ce qu'**il présente du macrogol 6000 dans une quantité de 1,0 à 10,0 % en poids par rapport au comprimé terminé.

8. Comprimé effervescent orodispersible selon l'une quelconque des revendications précédentes, **caractérisé en ce qu'**il présente du stéarate de magnésium dans une quantité de 0,05 à 0,5 % en poids par rapport au comprimé terminé.

9. Comprimé effervescent orodispersible selon l'une quelconque des revendications précédentes à utiliser pour le traitement de modifications inflammatoires de l'œsophage.

10. Comprimé effervescent orodispersible selon la revendication 9, la modification inflammatoire étant une œsophagite à éosinophiles.
